(19) Europäisches Patentamt
European Patent Office
Office européen des brevets

(11) Veröffentlichungsnummer: **0 129 832**
**B1**

(12) # EUROPÄISCHE PATENTSCHRIFT

(45) Veröffentlichungstag der Patentschrift: **19.04.89**

(51) Int. Cl.⁴: **C 07 D 235/32**

(21) Anmeldenummer: **84107026.1**

(22) Anmeldetag: **19.06.84**

(54) **Verfahren zur Verminderung von Nebenproduktanteilen bei der Herstellung von Carbendazim.**

(30) Priorität: **25.06.83 DE 3323024**

(43) Veröffentlichungstag der Anmeldung: **02.01.85 Patentblatt 85/1**

(45) Bekanntmachung des Hinweises auf die Patenterteilung: **19.04.89 Patentblatt 89/16**

(84) Benannte Vertragsstaaten: **AT BE CH DE FR GB IT LI NL SE**

(56) Entgegenhaltungen: **GB-A-1 348 460**

(73) Patentinhaber: **HOECHST AKTIENGESELLSCHAFT, Postfach 80 03 20, D-6230 Frankfurt am Main 80 (DE)**

(72) Erfinder: **Koch, Manfred, Dr., Kreuzheck 2, D-6239 Eppstein/Taunus (DE)**
Erfinder: **Maier, Thomas, Dr., Rauenthaler Weg 22, D-6000 Frankfurt am Main 71 (DE)**

EP 0 129 832 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Verfahren zur Verminderung von Nebenproduktanteilen bei der Herstellung von Carbendazim.

Carbendazim (2-Benzimidazol-carbaminsäuremethylester) ist ein wertvolles Fungizid mit einer großen

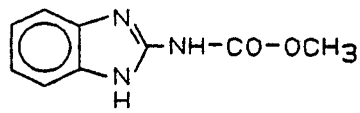

Carbendazim

Wirkungsbreite (H. Martin, Pesticide Manual (1977) S. 78, British Crop Protection Council). Es kann sowohl als technisches Fungizid wie insbesondere als systemisch wirkendes Pflanzenfungizid eingesetzt werden.

Die Herstellung von Carbendazim erfolgt bei allen gängigen Synthesen unter Verwendung von ortho-Phenylendiamin als Synthesebaustein. Dieses wird vorteilhaft mit Methylcyancarbamat gemäß DE-OS 1 688 557 und DE-OS 1 795 849 umgesetzt, wobei das Methylcyancarbamat direkt aus seiner Syntheselösung ohne Isolierung oder nach seiner Isolierung eingesetzt wird. Bei diesen Reaktionen entstehen dimere und polymere Kondensate des Phenylendiamins als Nebenprodukte, die zu unerwünschten Verfärbungen des Produktes führen und das Abwasser belasten. Die Nebenprodukte besitzen zum Teil auch toxische Eigenschaften.

Hinsichtlich der Verwendung von Carbendazim als technisches Fungizid für Schutzanstriche ist es deshalb von Bedeutung, die färbenden Begleitstoffe zu eliminieren. Ebenso ist es für den Einsatz in der Landwirtschaft wichtig, den Nebenproduktanteil des Carbendazims zu reduzieren.

Diese Aufgabe konnte durch vorliegende Erfindung Überraschenderweise dadurch gelöst werden, daß man bei der Synthese des Carbendazims ein Reduktionsmittel zusetzt.

Gegenstand der vorliegenden Erfindung ist daher ein Verfahren zur Verminderung von Nebenproduktanteilen bei der Synthese von Carbendazim ausgehend von o-Phenylendiamin und Methylcyancarbamat ($CH_3O$-CO-NH-CN) in Gegenwart von Wasser und protonischer Säure, dadurch gekennzeichnet, daß man dem Reaktionsgemisch ein Reduktionsmittel zusetzt, welches mit den übrigen Reaktionspartnern keine Reaktion eingeht und in wäßriger Lösung ausreichend stabil ist, wobei Ameisensäure ausgenommen ist. Als Syntheseverfahren zur Herstellung von Carbendazim kommen hierbei insbesondere die in den obengenannten deutschen Offenlegungsschriften beschriebenen Verfahren in Betracht. Es können jedoch auch andere bekannte Syntheseverfahren benutzt werden, bei denen erfindungsgemäß Reduktionsmittel zugesetzt werden.

Als Reduktionsmittel gemäß vorliegender Erfindung kommen übliche Reduktionsmittel infrage, wie Salze von Metallen in niedrigen Oxidationsstufen, z. B. Cu(I)-halogenide, Verbindungen von Nichtmetallen in niedrigen Oxidationsstufen, wie Verbindungen des Schwefels, Stickstoffs oder Phosphors, die reduzierende Eigenschaften besitzen, oder organische Verbindungen, die als Reduktionsmittel bekannt sind, wie Phloroglucin.

Zu beachten ist hierbei, daß das Reduktionsmittel so gewählt werden muß, daß es keine Reaktion mit den für die Herstellung von Carbendazim eingesetzten Reaktionspartnern eingeht; insbesondere darf die oxidierte Form des Reduktionsmittels nicht als Oxidationsmittel oder Katalysator für eine Oxidation des o-Phenylendiamins fungieren. Außerdem muß das Reduktionsmittel eine hinreichende Stabilität in wäßriger Lösung besitzen.

Besonders geeignete Reduktionsmittel sind Polysulfide wie Alkalidisulfide, Sulfide wie Alkali- oder Erdalkalisulfide, Hydrogensulfide wie Alkali- bzw. Erdalkalihydrogensulfide, Salze der Thioschwefelsäure, beispielsweise Alkali- oder Erdalkalisalze wie $Na_2S_2O_3$ oder Salze der Dithionigen Säure, beispielsweise Alkali- oder Erdalkalisalze wie $Na_2S_2O_4$.

Die Menge des verwendeten Reduktionsmittels ist nicht kritisch und liegt zweckmäßigerweise unter 10 Gew.-% bezogen auf eingesetztes o-Phenylendiamin oder unter 1 Gew.-% bezogen auf das Gesamtgewicht des Reaktionsgemisches. In der Regel liegt der Anteil des Reduktionsmittels zwischen 0,1 - 10 Gew.-%, bevorzugt zwischen 0,5 bis 5 Gew.-%, bezogen auf o-Phenylendiamin oder zwischen 0,01 und 1 Gew.-%, bevorzugt zwischen 0,05 bis 0,5 Gew.-% bezogen auf das gesamte Reaktionsgemisch.

Das Reduktionsmittel wird zweckmäßigerweise mit dem Methylcyancarbamat vorgegeben und der Reaktionspartner o-Phenylendiamin anschließend hinzugefügt.

Das Reduktionsmittel kann aber auch zusammen mit o-Phenylendiamin oder unmittelbar nach Zugabe des o-Phenylendiamins zugemischt werden. Eine späte Zugabe des Reduktionsmittels schmälert die gewünschte Reduzierung der Nebenproduktanteile.

Es ist ferner zweckmäßig, das Verfahren unter Inertgasatmosphäre, beispielsweise unter Stickstoff, auszuführen, um störende Einflüsse von Sauerstoff auf die Reaktion zu vermeiden.

Die übrigen Reaktionsparameter wie Temperatur und pH-Wert werden zweckmäßigerweise, wie in der DE-OS 1 795 848 beschrieben, einreguliert: Reaktionstemperatur 40 - 130°C; pH- Wert des Reaktionsgemisches 2,5 bis 4,5. Als protonische Säure wird zweckmäßigerweise wäßrige Salzsäure verwendet.

Das erfindungsgemäße Verfahren wird nachfolgend beispielhaft erläutert.

**Beispiel**

Zu einer wäßrigen Lösung von rohem Methylcyancarbamat (hergestellt aus 25 g Cyanamid, 57 g Chlorameisensäuremethylester und Natronlauge unter Stickstoff) wurde unter Stickstoff-Atmosphäre bei 25 - 35°C 0,9 g Natriumdithionit zugegeben, 10 min gerührt und mit 56,7 g o-Phenylendiamin versetzt. Man erwärmte auf 70°C und tropfte 89 ml 32 %-ige Salzsäure so zu, daß ein Temperaturbereich von 90 - 95°C und ein pH-Wert von 4 erreicht wurde. Nach 3 Stunden Reaktionszeit bei dieser Temperatur unter konstantem pH-Wert von 4 wurde das ausgefallene Produkt bei 70°C abfiltriert. Man wusch mit heißem Wasser chloridfrei und trocknete das Produkt im Vakuum. Man erhielt 91,6 g hochreinen 2-Benzimidazol-carbaminsäuremethylester, Fp. 315 - 318 (Zersetzung); Ausbeute bezogen auf o-Phenylendiamin: 91,5 %.

Das Produkt ist weiß und enthält keine färbenden Begleitstoffe.

Die Reinheit des Produkts bezüglich Dimeren bzw. Polymeren des o-Phenylendiamins läßt sich anhand des Gehaltes an 2,3-Diaminophenazin, ein Dimeres des o-Phenylendiamins, charakterisieren. So enthält der oben isolierte 2-Benzimidazol-carbaminsäuremethylester nur noch 0,8 - 0,9 ppm 2,3-Diaminophenazin. Das Filtrat weist nur einen Gehalt von 1 bis 4 ppm 2,3-Diaminophenazin auf und ist nur schwach gelb gefärbt.

**Patentansprüche**

1. Verfahren zur Verminderung von Nebenproduktanteilen bei der Synthese von Carbendazim

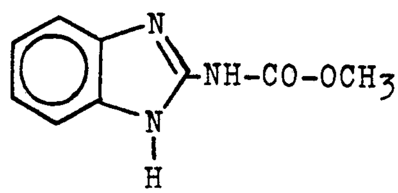

Carbendazim

ausgehend von o-Phenylendiamin und Methylcyancarbamat, in Gegenwart von Wasser und protonischer Säure, dadurch gekennzeichnet, daß man dem Reaktionsgemisch ein Reduktionsmittel zusetzt, welches mit den übrigen Reaktionspartnern keine Reaktion eingeht und in wäßriger Lösung ausreichend stabil ist, wobei Ameisensäure ausgenommen ist.

2. Verfahren gemäß Anspruch 1, dadurch gekennzeichnet, daß man das Reduktionsmittel zusammen mit dem Methylcyancarbamat vorgibt und anschließend das o-Phenylendiamin hinzufügt.

3. Verfahren gemäß Ansprüchen 1 und 2, dadurch gekennzeichnet, daß man als Reduktionsmittel ein Alkalioder Erdalkalisulfid, ein Alkali- oder Erdalkalihydrogensulfid, ein Salz der Thioschwefelsäure oder ein Salz der Dithionigen Säure verwendet.

4. Verfahren gemäß einem oder mehreren der Ansprüche 1 bis 3, dadurch gekennzeichnet, daß man als Reduktionsmittel ein Alkali- oder Erdalkalisalz der Thioschwefelsäure oder der Dithionigen Säure einsetzt.

5. Verfahren gemäß einem oder mehreren der Ansprüche 1 bis 4, dadurch gekennzeichnet, daß $Na_2S_2O_3$ oder $Na_2S_2O_4$ als Reduktionsmittel eingesetzt wird.

6. Verfahren gemäß einem oder mehreren der Ansprüche 1 bis 5, dadurch gekennzeichnet, daß das Reduktionsmittel in einer Menge von 0,1 - 10 Gewichtsprozent bezogen auf eingesetztes o-Phenylendiamin verwendet wird.

7. Verfahren gemäß einem oder mehreren der Ansprüche 1 bis 6, dadurch gekennzeichnet, daß das Reduktionsmittel in einer Menge von 0,5 bis 5 Gew.-% bezogen auf eingesetztes o-Phenylendiamin verwendet wird.

8. Verfahren gemäß einem oder mehreren der Ansprüche 1 bis 7, dadurch gekennzeichnet, daß das Reduktionsmittel in einer Menge von 0,01 bis 1,0 Gew.-% bezogen auf das gesamte Reaktionsgemisch verwendet wird.

9. Verfahren gemäß einem oder mehreren der Ansprüche 1 bis 8, dadurch gekennzeichnet, daß man es unter Inertgas-Atmosphäre durchführt.

**Claims**

1. A process for reducing the proportions of by products in the synthesis of carbendazim

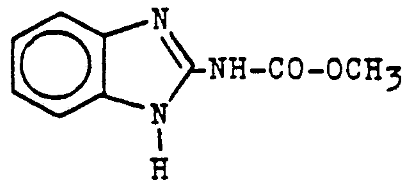

carbendazim

starting from o-phenylenediamine and methyl cyanocarbamate, in the presence of water and protonic acid, which comprises adding a reducing agent to the reaction mixture which does not enter into any reaction with the other reactants and is sufficiently stable in aqueous solution, with the exception of formic acid.

2. The process as claimed in claim 1, wherein the reducing agent is initially introduced together with the methyl cyanocarbamate, and then the o-phenylenediamine is added.

3. The process as claimed in claims 1 and 2, wherein the reducing agent used is a sulfide of an alkali metal or alkaline earth metal, a hydrogensulfide of an alkali metal or alkaline earth metal, a salt of thiosulfuric acid or a salt of dithionous acid.

4. The process as claimed in one or more of claims 1 to 3, wherein the reducing agent used is a salt of thiosulfuric acid or of dithionous acid with an alkali metal or alkaline earth metal.

5. The process as claimed in one or more of claims 1 to 4, wherein $Na_2S_2O_3$ or $Na_2S_2O_4$ is used as the reducing agent.

6. The process as claimed in one or more of claims 1 to 5, wherein the reducing agent is used in an amount of 0.1 - 10 percent by weight relative to o-phenylenediamine used.

7. The process as claimed in one or more of claims 1 to 6, wherein the reducing agent is used in an amount of 0.5 to 5 percent by weight relative to o-phenylenediamine used.

8. The process as claimed in one or more of claims 1 to 7, wherein the reducing agent is used in an amount of 0.01 to 1.0 percent by weight relative to the total reaction mixture.

9. The process as claimed in one or more of claims 1 to 8, which is carried out under an atmosphere of inert gas.

**Revendications**

1. Procédé pour diminuer les proportions de produits secondaires lors de la synthèse du carbendazime:

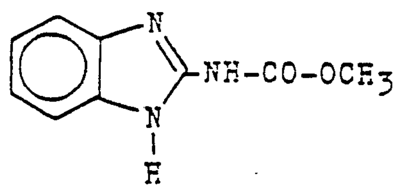

Carbendazime

à partir de l'o-phénylène-diamine et du cyanocarbamate de méthyle, en présence d'eau et d'un acide protonique, procédé caractérisé en ce qu'on ajoute au mélange réactionnel un réducteur qui ne donne pas de réactions avec les autres partenaires réactionnels et qui est suffisamment stable en solution aqueuse à l'exception de l'acide formique.

2. Procédé selon la revendication 1 caractérisé en ce qu'on met d'abord en place le réducteur avec le cyanocarbamate de méthyle et en ce qu'on ajoute ensuite l'o-phénylène-diamine.

3. Procédé selon l'une des revendications 1 et 2, caractérisé en ce qu'on utilise, comme réducteur, un sulfure de métal alcalin ou de métal alcalino-terreux, un hydrogénosulfure de métal alcalin ou de métal alcalinoterreux, un sel de l'acide thiosulfurique ou un sel de l'acide dithioneux.

4. Procédé selon l'une quelconque des revendications 1 à 3, caractérisé en ce qu'on utilise, comme réducteur, un sel de métal alcalin ou de métal alcalino-terreux de l'acide thiosulfurique ou de l'acide dithioneux.

5. Procédé selon l'une quelconque des revendications 1 à 4, caractérisé en ce qu'on utilise, comme réducteur, $Na_2S_2O_3$ ou $Na_2S_2O_4$.

6. Procédé selon l'une quelconque des revendications 1 à 5, caractérisé en ce que le réducteur est utilisé en une quantité de 0,1 à 10 % en poids par rapport à l'o-phénylène-diamine mise en jeu.

7. Procédé selon l'une quelconque des revendications 1 à 6, caractérisé en ce que le réducteur est utilisé en une quantité de 0,5 à 5 % en poids par rapport à l'o-phénylène-diamine mise en jeu.

8. Procédé selon l'une quelconque des revendications 1 à 7, caractérisé en ce que le réducteur est utilisé en

une quantité de 0,01 à 1,0 % en poids par rapport au mélange réactionnel total.

9. Procédé selon l'une quelconque des revendications 1 à 8, caractérisé en ce qu'il est exécuté sous un gaz inerte.